# FASCICULE DE BREVET EUROPEEN

(11) **EP 1 640 445 B1**
(45) Date de publication et mention de la délivrance du brevet: **02.02.2011**
(21) Numéro de dépôt: 05292003.0
(22) Date de dépôt: 27.09.2005
(51) Int. Cl.: C12M 1/10

(54) **Tambour destiné à une installation pour la fermentation aérobie de déchets**
Trommel für eine Anlage zur aeroben Fermentierung von Abfällen
Drum for an installation for aerobic fermentation of waste

(30) Priorité: 27.09.2004 FR 0452164
(43) Date de publication de la demande: 29.03.2006
(73) Titulaire: Vinci Environnement, 92500 Rueil Malmaison (FR)
(72) Inventeur: Felidj, Karim, 92300 Levallois (FR); Seutin, Hugues, 78150 Le Chesnay (FR)
(74) Mandataire: Enderlin, Eric André

(56) Documents cités:
- FR-A- 2 746 410
- FR-A- 2 796 865
- FR-A- 2 832 780

## Description

L'invention concerne un tambour pour la fermentation aérobie de déchets, ainsi qu'une installation comprenant un tel tambour.

De tels tambours comprennent généralement une enveloppe cylindrique, entraînée en rotation autour de son axe, dans laquelle les déchets fermentent sous l'action de micro-organismes aérobies, un dispositif de ventilation étant prévu à cet effet pour créer une circulation d'air à l'intérieur du tambour. Il est notamment connu de relier un ventilateur à l'extrémité amont de l'enveloppe, des orifices étant prévus à la partie aval de l'enveloppe pour permettre la circulation d'air à l'intérieur du tambour, par aspiration ou refoulement induit par le ventilateur.

Le document FR 2 746 410 décrit un tambour comprenant une paroi transversale formant bouclier de sortie, dans laquelle est ménagée une ouverture. Cette ouverture a une double fonction : d'une part, elle permet le déchargement des déchets fermentés en phase de déchargement et, d'autre part, elle permet le passage d'air de ventilation en phase de non déchargement.

Cette structure présente un certain nombre de limites. Tout d'abord, elle nécessite l'emploi de moyens d'accouplement / désaccouplement du bouclier de sortie et de l'enveloppe, selon que l'on est en phase de déchargement ou non. Par ailleurs, le fait que la même ouverture serve à la fois au déchargement des déchets et à la ventilation ne permet pas d'obtenir une circulation d'air optimale dans le tambour. La performance du tambour en termes de capacité de fermentation des déchets n'est donc pas pleinement satisfaisante.

Le document FR 2 832 780 décrit un tambour dont l'enveloppe cylindrique est pourvue d'évents aptes à être ouverts ou fermés par un dispositif comprenant une plaque mobile. Toutefois, une telle disposition des évents pose différents problèmes.

En effet, l'air est introduit par les évents à l'intérieur de l'enveloppe de façon radiale. Ce mode d'injection d'air ne permet pas une répartition et une circulation de l'air optimales dans le tambour. Or, la qualité de la ventilation à l'intérieur de l'enveloppe est cruciale, car elle conditionne l'activité des micro-organismes assurant la fermentation, et donc l'efficacité du tambour.

En outre, il existe un risque que les déchets viennent colmater les évents lors de la rotation du tambour.

Le document FR 2 796 865 décrit un tambour pour la fermentation comportant une enveloppe destinée à accueillir des déchets et pouvant être mise en rotation autour de son axe longitudinal. Le tambour comporte également une paroi transversale fixe d'arrivée des déchets à traiter situées à l'entrée du tambour et immobile par rapport à l'enveloppe rotative et une paroi transversale de sortie des déchets traités situées à la sortie du tambour et tournant simultanément avec l'enveloppe rotative. La paroi transversale de sortie comporte une trappe d'évacuation des déchets et un orifice de ventilation distinct de la trappe. Une grille est disposée contre l'orifice de ventilation.

L'invention a pour but de résoudre ces problèmes.

A cet effet, et selon un premier aspect, l'invention concerne un tambour destiné à une installation pour la fermentation aérobie de déchets, comprenant :
- une enveloppe dans laquelle les déchets à traiter sont destinés à être introduits, ladite enveloppe présentant une extrémité amont, une extrémité aval, et un axe nominal, ladite enveloppe étant destinée à être entraînée en rotation autour de son axe lors de la fermentation ;
- un bouclier de sortie comprenant une paroi transversale disposée en regard de l'extrémité aval de l'enveloppe ;
- une trappe prévue dans la paroi transversale, apte à permettre l'évacuation des déchets fermentés.

Selon une définition générale de l'invention, la paroi transversale comprend au moins un orifice de ventilation distinct de la trappe, ledit orifice étant disposé de sorte à permettre une circulation axiale d'air à travers lui, entre l'intérieur et l'extérieur de l'enveloppe.

Le tambour comprend un dispositif d'obturation de l'orifice de ventilation, ledit dispositif étant agencé pour pouvoir être déplacé entre une position de fermeture, dans laquelle l'orifice de ventilation est obturé, et une position d'ouverture, dans laquelle l'orifice de ventilation est dégagé.

Par exemple, le dispositif d'obturation est maintenu dans sa position de fermeture par des moyens de rappel, ledit dispositif d'obturation étant apte à être déplacé vers sa position d'ouverture sous l'action d'une force d'appui exercée à l'encontre de la force des moyens de rappel.

Le dispositif d'obturation peut comprendre au moins un volet disposé de sorte à pouvoir obturer, respectivement dégager l'orifice, lorsque le dispositif d'obturation est en position de fermeture, respectivement d'ouverture, et au moins une tige solidaire du volet, l'ensemble volet - tige étant monté sur la face extérieure de la paroi transversale de façon à pouvoir coulisser sensiblement radialement.

Selon un deuxième aspect, l'invention concerne une installation pour la fermentation aérobie de déchets, comprenant un tambour tel que précédemment décrit, des moyens de support du tambour agencés pour que l'axe nominal de l'enveloppe du tambour soit sensiblement horizontal ou légèrement incliné par rapport à l'horizontale, et au moins un système d'entraînement apte à permettre l'entraînement du tambour en rotation autour de son axe.

L'installation comprend par exemple une rampe en arc de cercle fixe par rapport au sol, disposée à l'extérieur de l'enveloppe, de façon sensiblement concentrique à l'enveloppe, ladite rampe étant apte à coopérer avec un galet prévu sur le dispositif d'obturation, de sorte à provoquer le déplacement du dispositif d'obturation de la position de fermeture à la position d'ouverture par appui du galet contre la rampe.

La rampe est agencée pour coopérer avec le galet lorsque l'orifice de ventilation n'est pas disposé en vis à vis des déchets présents dans le tambour. A cet effet, la rampe est par exemple disposée au-dessus du tambour, et s'étend au moins sur un quart de la périphérie du tambour, entre un plan vertical passant par l'axe du tambour et un plan horizontal passant par l'axe du tambour.

En outre, la rampe peut comporter une zone centrale sensiblement circulaire, ainsi qu'une zone d'entrée et une zone de sortie aptes à guider le déplacement du dispositif d'obturation entre ses positions d'ouverture et de fermeture.

Selon une réalisation possible, l'installation comprend un dispositif de ventilation permettant la circulation d'air à l'intérieur du tambour, le dispositif de ventilation comprenant au moins un ventilateur relié à l'extrémité amont de l'enveloppe du tambour, ledit ventilateur étant agencé pour aspirer et/ou refouler l'air, de sorte que l'air soit introduit, respectivement évacué, au niveau de l'extrémité aval de l'enveloppe de façon sensiblement axiale à travers l'orifice de ventilation.

Par exemple, le système d'entraînement comprend un pignon entraîné en rotation autour d'un axe principal sensiblement parallèle à l'axe nominal du tambour et une couronne dentée solidaire du tambour, ledit pignon étant disposé de sorte à venir engrener la couronne pour commander la rotation du tambour autour de son axe nominal, le pignon étant en outre monté mobile par rapport à un premier axe de flottement distinct de l'axe principal du pignon, ledit montage étant agencé pour permettre au moins la rotation libre du pignon autour dudit premier axe de flottement.

Le pignon peut en outre être monté mobile par rapport à un deuxième axe de flottement distinct de l'axe principal du pignon et du premier axe de flottement, ledit montage étant agencé pour permettre au moins la rotation libre du pignon autour dudit deuxième axe de flottement.

Les autres caractéristiques de l'invention résultent de la description qui suit d'un mode de réalisation, description effectuée en référence aux figures annexées dans lesquelles :
- la figure 1 est une représentation schématique partielle d'une installation pour la fermentation aérobie de déchets comprenant un tambour selon l'invention ;
- la figure 2 est une vue schématique en élévation du bouclier de sortie du tambour de la figure 1 ;
- la figure 3 est une vue en coupe selon un plan axial vertical du tambour de la figure 1, dans la zone du dispositif amont de guidage en rotation ;
- la figure 4 est une vue partielle en coupe du tambour selon la ligne AA de la figure 1 ;
- la figure 5 est une représentation schématique en coupe du tambour selon la ligne BB de la figure 1 ;
- la figure 6 est une vue similaire à celle de la figure 5, illustrant plus spécifiquement le système d'entraînement en rotation ;
- la figure 7 est vue similaire à celle de la figure 6, représentant de façon schématique la position des éléments constitutifs du système d'entraînement en rotation après une déformation radiale du tambour.

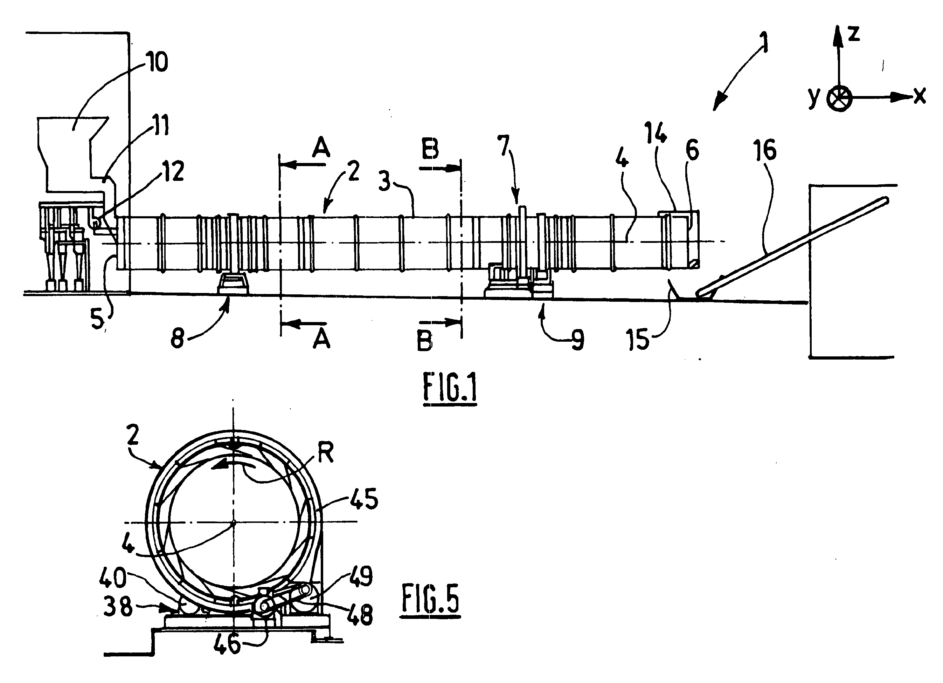

On se réfère tout d'abord à la figure 1 qui représente une installation 1 pour la fermentation aérobie de déchets.

L'installation 1 comprend tout d'abord un tambour 2, lui-même comportant une enveloppe 3 sensiblement cylindrique, d'axe nominal 4, ainsi qu'un bouclier d'entrée comprenant une paroi transversale amont 5, et un bouclier de sortie comprenant une paroi transversale aval 6, les parois transversales étant sensiblement en forme de disque. L'enveloppe 3 est réalisée en métal. Elle peut atteindre 40 à 50 m de longueur, et son diamètre peut être de l'ordre de 3 à 5 m.

Une localisation éloignée de l'axe 4 sera dite « extérieure », par opposition à une localisation plus proche de l'axe 4, dite « intérieure ». On définit l'axe X comme l'axe parallèle à l'axe nominal 4, orienté vers l'aval, l'axe Z comme l'axe vertical ascendant, et l'axe Y tel que (X, Y, Z) forme un repère orthogonal.

Le tambour 2 est disposé sur le sol de sorte que son axe 4 soit sensiblement horizontal, ou légèrement incliné par rapport à l'horizontale, de la paroi transversale amont 5 vers la paroi transversale aval 6, d'un angle inférieur à 10°, voire inférieur à 5°.

L'installation 1 comprend en outre un système d'entraînement 7 en rotation du tambour 2 autour de l'axe 4, disposé environ aux deux tiers de l'enveloppe 3 à partir de la paroi transversale amont 5. Le sens de rotation est indiqué sur les figures par les flèches R. Deux dispositifs de guidage en rotation, respectivement amont 8 et aval 9 sont également prévus.

L'installation 1 comprend des moyens de chargement des déchets à traiter, reliés à l'extrémité amont de l'enveloppe 3 du tambour 2. Ces moyens comportent une trémie de chargement 10, ainsi qu'un conduit 11 d'amenée des déchets depuis la trémie 10 vers l'intérieur du tambour 2, via un orifice ménagé dans la paroi transversale amont 5.

En entrée du tambour est également prévu un ventilateur 12, connecté à l'orifice de la paroi transversale amont 5 par lequel les déchets sont introduits, le ventilateur 12 étant apte à provoquer une circulation d'air à l'intérieur du tambour 2, comme on le verra plus loin.

Une masse déterminée de déchets est introduite à des moments choisis dans le tambour 2, via la paroi transversale amont 5. Pendant cette phase de chargement, le ventilateur 12 n'est pas en fonctionnement.

Les déchets sont brassés du fait du mouvement de rotation du tambour 2, et déplacés vers le bouclier de sortie, notamment par la poussée des nouveaux déchets introduits dans le tambour 2. Des couteaux peuvent être prévus à l'intérieur de l'enveloppe 3 pour favoriser le brassage et la séparation des déchets.

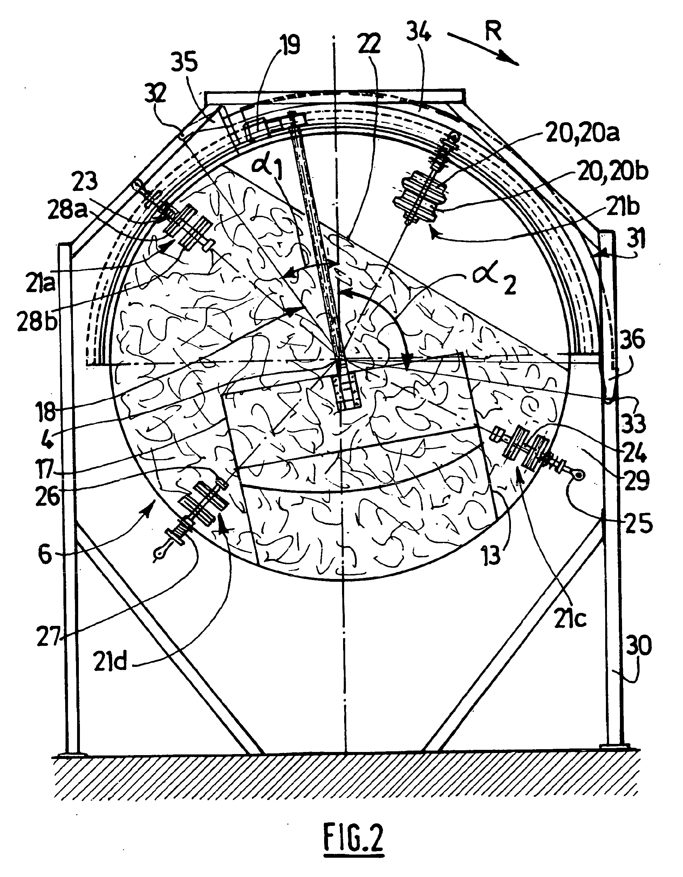

Pendant leur séjour dans le tambour, d'environ 2 à 3 jours, les déchets sont progressivement fermentés par des micro-organismes aérobies dont l'activité est augmentée par une circulation d'air appropriée. Les déchets présents dans le tambour 2 occupent par exemple les deux tiers du volume du tambour. Du fait de la rotation du tambour 2, les déchets forment en moyenne une surface libre 22 inclinée par rapport à l'horizontale (voir figure 2). Dans la réalisation représentée, la surface libre 22 est inclinée d'un angle voisin de 45°. Bien entendu, cet angle dépend notamment de la nature des déchets et de la vitesse de rotation du tambour 2.

La paroi transversale aval 6 comporte une trappe 13 permettant l'évacuation des déchets ainsi fermentés. En sortie, ces déchets se présentent sous forme d'amas de taille relativement importante. Afin de réduire la taille de ces amas, une pièce cylindrique 14 solidaire du tambour 2 est prévue. Cette pièce 14, ouverte à ses extrémités amont et aval, présente une surface latérale cylindrique munie d'orifices, de sorte à former un crible.

Enfin, l'installation 1 comprend des moyens d'évacuation des déchets fermentés, situés en aval du tambour 2. Ces moyens comprennent par exemple une trémie 15 de récupération et un transporteur à bande 16 apte à acheminer les déchets vers une autre unité.

On décrit maintenant la paroi transversale aval 6 du bouclier de sortie du tambour 2, en référence à la figure 2.

La trappe 13 d'évacuation des déchets fermentés est située dans la zone périphérique de la paroi transversale 6 du bouclier de sortie. Dans la réalisation représentée, la trappe 13 présente sensiblement la forme d'un rectangle dont un côté est arrondi de sorte à épouser localement la forme circulaire du bord de la paroi transversale 6. La hauteur de la trappe 13, de son côté arrondi à son côté opposé, est de l'ordre d'un demi rayon de la paroi transversale 6, et la distance entre les deux autres côtés de la trappe 13 est voisine du double de la hauteur.

Un panneau 17 d'obturation de la trappe 13 est prévu, de sorte que l'on puisse choisir de décharger les déchets ou non. Par exemple, il peut être prévu de maintenir la trappe 13 fermée pendant la nuit, et de l'ouvrir dans la journée.

Le panneau 17 est monté mobile entre une position de fermeture, dans laquelle il obture la trappe 13, et une position d'ouverture, dans laquelle la trappe 13 est dégagée. A cet effet est prévu un dispositif 18 de déplacement du panneau 17, actionné par un moteur 19 embarqué sur le tambour 2. Le dispositif 18 peut consister en un vérin, un ensemble manchon taraudé - vis filetée, ou tout autre mécanisme approprié.

La paroi transversale 6 comprend également une pluralité d'orifices 20 de ventilation, destinés à permettre l'entrée d'air dans le tambour 2, et la circulation de cet air sensiblement parallèlement à l'axe 4, grâce à l'aspiration provoquée par le ventilateur 12.

La paroi transversale 6 présente au moins deux ensembles d'au moins un orifice de ventilation, lesdits ensembles étant répartis sur la surface de ladite paroi transversale et espacés les uns des autres sensiblement régulièrement. Au moins un ensemble comprend au moins deux orifices situés sensiblement sur un même rayon, dans la zone périphérique de la paroi transversale.

Dans la réalisation représentée, les orifices 20 sont répartis sur la surface de la paroi transversale 6 en quatre ensembles 21 de deux orifices 20 chacun, respectivement un premier ensemble 21a, un deuxième ensemble 21b, un troisième ensemble 21c et un quatrième ensemble 21d.

Dans un ensemble donné 21, les orifices 20 sont situés sensiblement sur un même rayon de la paroi transversale 6, dans la zone périphérique de ladite paroi 6. Un premier orifice 20a, dit extérieur, est par exemple situé à une distance du bord circulaire de la paroi transversale 6 de l'ordre de 10 à 20 cm, un deuxième orifice 20b, dit intérieur, étant situé à une distance du bord circulaire de la paroi transversale 6 de l'ordre de 30 à 40 cm. Les orifices extérieurs 20a des quatre ensembles 21 son situés sensiblement sur un même cercle centré sur l'axe 4. De même, les orifices intérieurs 20b des quatre ensembles 21 son situés sensiblement sur un même cercle, de diamètre plus petit, centré sur l'axe 4.

Les troisième et quatrième ensembles 21c, 21d sont situés de part et d'autre de la trappe 13, et écartés angulairement de 100° environ. L'écart angulaire entre les premier et quatrième ensembles 21a, 21d d'une part, et entre les deuxième et troisième ensembles 21 b, 21 c d'autre part, est d'environ 95°.

Les orifices 20 présentent sensiblement la même forme. Ils sont rectangulaires, de hauteur (selon un rayon) de l'ordre de 5 à 15 cm, et de largeur de l'ordre de 25 à 40 cm. Les deux orifices 20a, 20b d'un même ensemble sont espacés radialement d'une distance de l'ordre de 10 cm.

Un dispositif d'obturation 23 est prévu, pour chaque ensemble 21, pour permettre l'ouverture et la fermeture contrôlées des orifices 20.

Chaque dispositif d'obturation 23 comprend tout d'abord une tige 24 disposée à l'extérieur du tambour 2, en regard, sensiblement parallèlement et à proximité immédiate de la paroi transversale 6, ladite tige 24 s'étendent sensiblement radialement. A l'extrémité extérieure de la tige 24, située au-delà du bord circulaire de la paroi transversale 6, est fixé un galet 25 d'axe sensiblement parallèle à l'axe 4 du tambour 2. En outre, la tige 24 est guidée en translation radiale par des manchons de coulissement 26, 27 situés respectivement à la partie extrême intérieure et à la partie extrême extérieure de la tige 24.

La tige 24 porte deux volets 28a, 28b disposés sensiblement parallèlement à la paroi transversale 6, et à proximité immédiate de celle-ci. La position des volets sur la tige 24, leur forme et leurs dimensions sont prévues pour permettre d'une part l'obturation des orifices 20, et d'autre part le dégagement de ces orifices 20.

Par exemple, les volets 28a, 28b présentent une forme rectangulaire sensiblement identique à la forme des orifices 20, mais de dimensions légèrement plus grandes, de sorte à bien recouvrir les orifices 20 pour assurer leur obturation. De plus, l'écartement entre les deux volets 28a, 28b est adapté à l'écartement entre les deux orifices 20a, 20b d'un même ensemble 21.

Un ressort 29, logé dans le manchon 27, exerce une force radiale orientée vers l'extérieur du tambour 2, tendant à maintenir la tige 24 et les volets 28a, 28b associés dans une position d'obturation des orifices 20.

L'installation 1 comporte en outre une charpente 30 dont une partie s'étend au-dessus du tambour 2 et soutient une rampe 31.

La rampe 31 présente la forme d'un arc de cercle, placé à l'extérieur de l'enveloppe 3, de façon sensiblement concentrique. La rampe 31 est disposée de sorte à s'étendre en vis à vis de la surface libre 22 au moins d'une extrémité longitudinale à l'autre de cette surface libre 22. Par exemple, la rampe 31 s'étend sur environ 130°, entre une première extrémité 32 située en amont de la verticale, à un angle α1 d'environ 20°, et une deuxième extrémité 33 située en aval de la verticale, à un angle α2 d'environ 110°, par rapport au sens de rotation R du tambour 2. Dans cette configuration la surface libre 22 forme sensiblement une corde de la rampe 31.

La rampe 31 comporte une zone centrale 34, dont la face en regard du tambour 2 est sensiblement circulaire, ainsi qu'une zone d'entrée 35 et une zone de sortie 36 dont les faces en regard du tambour 2 sont inclinées de manière que les extrémités 32, 33 de la rampe 31 soient situées à une distance radiale du tambour 2 plus importante que la zone centrale 34.

Lors de la rotation du tambour 2, le galet 25 d'un dispositif d'obturation 23 vient au contact de la zone d'entrée 35 de la rampe 31 et se déplace vers la zone centrale 34, provoquant de ce fait le coulissement de la tige 24 radialement, vers l'axe 4 du tambour 2, en comprimant le ressort 29.

La distance radiale entre la rampe 31 et le tambour 2 est prévue pour permettre la coopération du galet 25 avec la rampe 31, ainsi qu'un déplacement d'amplitude déterminée de la tige 24 pour que les volets 28a, 28b se déplacent vers l'axe 4, dégageant ainsi les orifices 20a, 20b. Le déplacement de la tige 24 est tel que, notamment, le volet extérieur 28a se situe alors entre les orifices extérieur 20a et intérieur 20b, de sorte que ledit volet a dégagé l'orifice extérieur 20a sans obturer l'orifice intérieur 20b.

La rotation du tambour se poursuivant, le galet 25 arrive en regard de la zone de sortie 36 de la rampe 31, le ressort 29 ramenant alors progressivement la tige 24 et les volets 28a, 28b vers la périphérie de la paroi transversale 6 jusqu'à la position d'obturation des orifices 20, lorsque le galet 25 n'est plus en contact avec la rampe 31.

De ce fait, les orifices 20 sont dégagés lorsqu'ils ne sont pas en regard des déchets présents dans le tambour 2, permettant ainsi l'entrée d'air axialement par l'action du ventilateur 12, et les orifices 20 sont obturés lorsqu'ils sont en regard des déchets, de sorte à empêcher un déchargement intempestif du tambour 2.

La circulation axiale des déchets depuis le bouclier d'entrée vers le bouclier de sortie, et la circulation axiale de l'air de l'aval vers l'amont ou dans le sens inverse conduit à une activité de fermentation particulièrement performante.

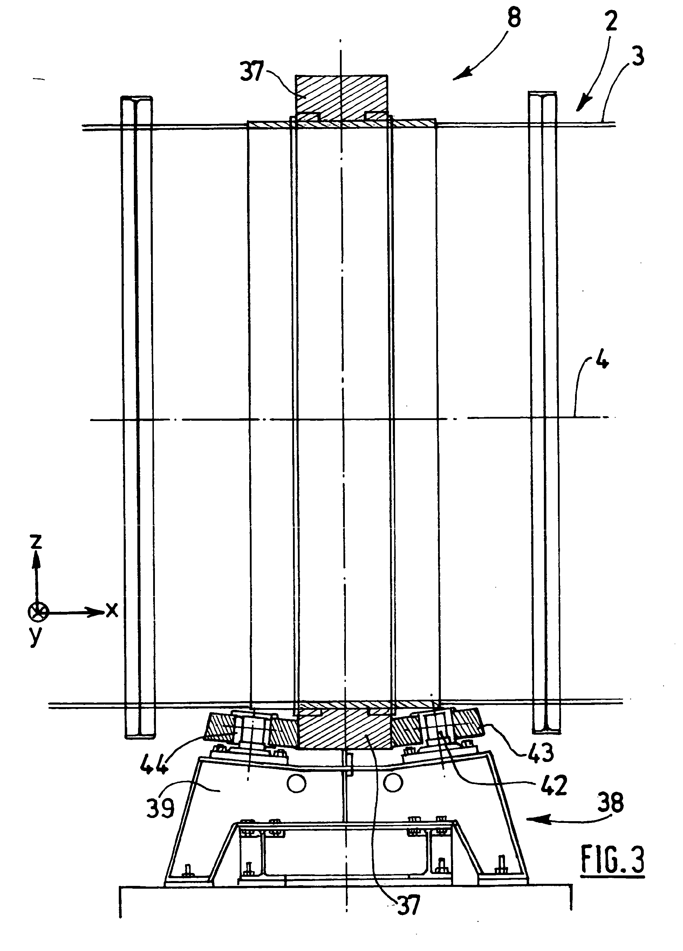

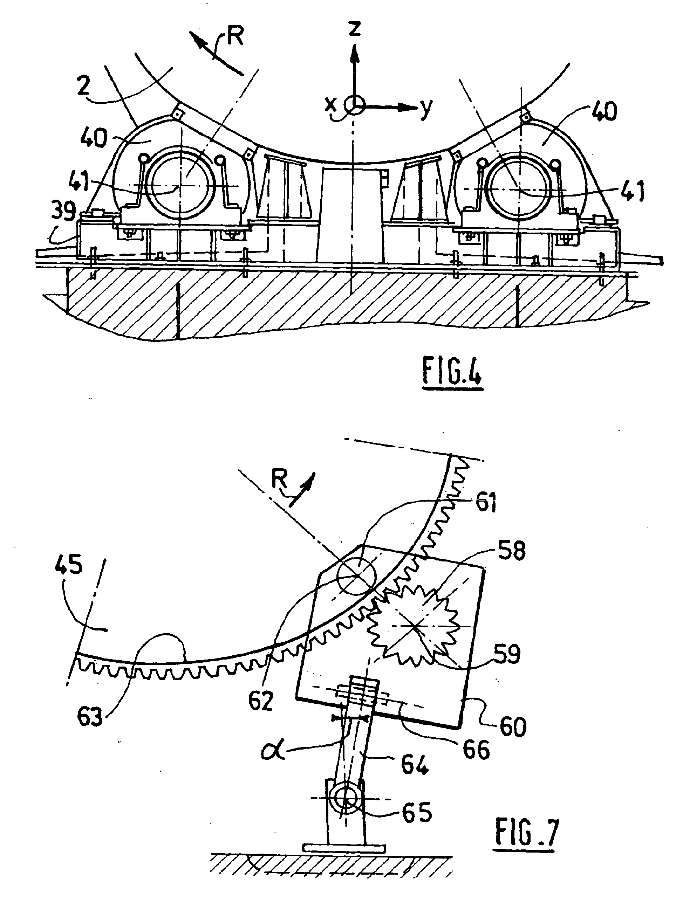

On décrit à présent le dispositif amont 8 de guidage en rotation du tambour 2, en référence aux figures 3 et 4. Il est à noter que le dispositif aval de guidage en rotation 9 peut être identique ou non.

Le dispositif amont 8 de guidage en rotation comprend tout d'abord un organe de maintien, tel qu'une ceinture 37, solidaire de l'enveloppe 3 du tambour 2.

En outre, un dispositif de support 38 du tambour 2 est fixé au sol. Le dispositif de support 38 comprend un bâti 39 auquel sont associés quatre galets 40 d'axe 41 parallèle à l'axe 4, disposés sensiblement aux coins d'un rectangle. Le tambour repose sur les galets 40 qui autorisent sa rotation autour de l'axe 4. Sur la face supérieure du bâti 39 sont fixés des organes saillants 42 associés à une pièce 43 solidaire de la ceinture 37 via des roulements 44, de sorte à permettre, localement, une certaine amplitude de rotation du tambour 2 par rapport au bâti 39 autour de l'axe Y.

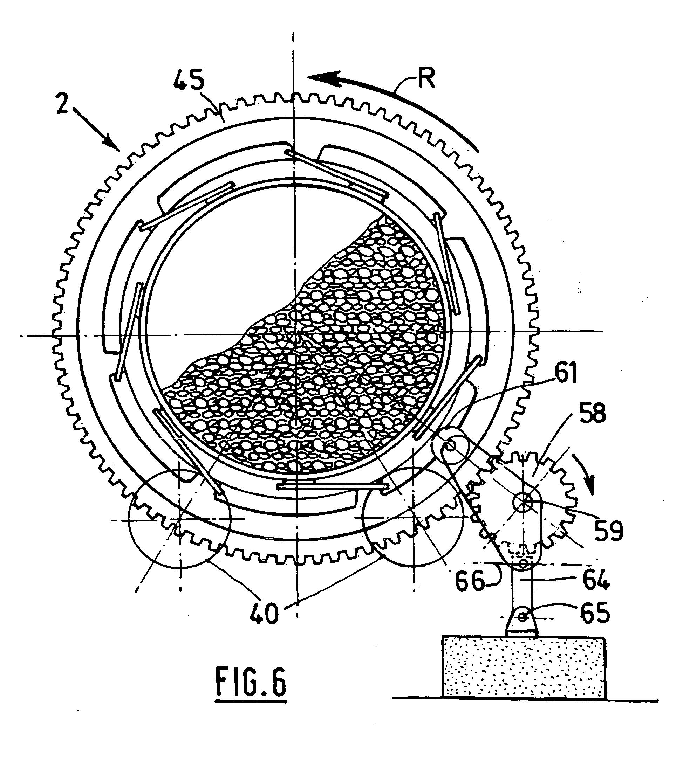

On se rapporte maintenant aux figures 5 et 6 qui illustrent le système d'entraînement 7 en rotation du tambour 2. Ce système d'entraînement 7 est décrit « au repos », c'est-à-dire en l'absence d'une dilatation du tambour 2.

Le système d'entraînement 7 comprend un organe d'entraînement tel qu'une couronne dentée 45 solidaire du tambour 2, d'axe nominal sensiblement confondu avec l'axe 4. La couronne dentée 45 est apte à coopérer avec un dispositif moteur pour l'entraînement en rotation du tambour 2.

Le dispositif moteur comprend un moteur 46 d'axe sensiblement parallèle à l'axe 4, dont la rotation est transmise via une courroie 48 à un premier dispositif réducteur. La courroie 48 est située dans un plan sensiblement orthogonal à l'axe 4, la premier dispositif réducteur étant plus éloigné du tambour 2 que le moteur 46. Le moteur 46 et le premier dispositif réducteur sont montés sur un support fixé au sol.

La sortie du premier dispositif réducteur est connectée à l'entrée d'un deuxième dispositif réducteur via un dispositif de liaison. Le dispositif de liaison comprend par exemple une barre de transmission sensiblement parallèle à l'axe 4, dont l'extrémité amont est associée à la sortie du premier dispositif réducteur par un premier cardan, et dont l'extrémité aval est associée à l'entrée du deuxième dispositif réducteur par un deuxième cardan et des moyens de coulissement selon l'axe X. Les moyens de coulissement sont formés par un premier cylindre d'axe X apte à coulisser selon son axe dans un deuxième cylindre concentrique, l'ensemble étant protégé par une membrane souple de type soufflet.

Le deuxième dispositif réducteur comprend un manchon denté solidaire du deuxième cylindre, agencé pour venir en prise avec un pignon 58 d'axe principal 59 sensiblement parallèle à l'axe nominal 4 du tambour 2. Le pignon 58 est donc entraîné en rotation autour de son axe principal 59, par le moteur 46.

Le pignon 58 est situé entre le tambour 2 et le deuxième dispositif réducteur, et est disposé de sorte à venir engrener la couronne 45 pour commander la rotation du tambour 2 autour de son axe nominal 4. Le pignon 58 est en partie logé dans un carter 60 auquel sont associés en rotation deux galets 61 d'axe 62 sensiblement parallèle à l'axe X. Les galets 61 sont agencés pour maintenir la couronne dentée 45 en prise avec le pignon 58. A cet effet, ils sont disposés aux extrémités amont et aval de la couronne 45, contre une face 63 de la dite couronne 45 dirigée vers l'axe 4.

Le carter 60 est fixé au sol par l'intermédiaire d'une barre 64 sensiblement verticale dont l'extrémité inférieure est associée au sol de façon rotative autour d'un premier axe de flottement 65, sensiblement parallèle à l'axe X, et dont l'extrémité supérieure est associée au carter 60 de façon rotative autour d'un deuxième axe de flottement 66 sensiblement parallèle à l'axe Y. Le deuxième axe de flottement 66 est situé sensiblement dans un plan (Y, Z) médian de la couronne 45. En outre, la longueur de la barre 64 est choisie, en fonction du support du moteur 46 et du premier dispositif réducteur, pour que la barre de transmission soit sensiblement horizontale.

Lorsque le tambour se dilate, du fait de l'augmentation de température provoquée par la fermentation des déchets, le pignon 58 est apte à « suivre » la déformation du tambour 2, au niveau de la couronne dentée 45, grâce aux axes de flottement 65, 66, de sorte que les dents du pignon 58 et de la couronne 45 restent toujours en prise. Ce déplacement du pignon 58 est « libre », c'est-à-dire provoqué par le déplacement de la couronne 45, par opposition à la rotation du pignon 58 autour de son axe principal 59, qui est motorisée.

Dans la réalisation représentée, le pignon 58 est monté mobile en rotation autour des axes de flottement 65, 66. En variante, le pignon 58 pourrait être monté sur des patins élastomères aptes à autoriser une telle rotation.

La figure 7 illustre de façon schématique la déformation radiale du tambour 2, conduisant à l'augmentation du diamètre de la couronne 45. En conséquence, le pignon 58, maintenu contre la couronne 45 par le galet 61, se déplace en rotation autour de l'axe 65, d'un angle α. Ainsi, malgré la dilatation radiale du tambour 2, le pignon 58 reste en prise avec la couronne 45.

Le tambour 2 est également susceptible de se dilater axialement. Le tambour 2 étant maintenu à ses parties extrêmes par les dispositifs de guidage en rotation 8, 9, l'allongement axial du tambour 2 conduit à une déformation de type fléchissement, l'axe 4 n'étant alors plus parallèle à l'axe X, notamment dans la zone du système d'entraînement 7.

Les dents de la couronne 45 et du pignon 58 ne sont alors plus parfaitement alignées, le parallélisme entre l'axe principal 59 du pignon 58 et l'axe 4 pouvant être localement rétabli par la rotation du pignon 58 autour du deuxième axe de flottement 66.

Les cardans permettent d'assurer la transmission du moteur 46 au pignon 58 même lorsque les axes du moteur et du pignon ne sont pas parfaitement alignés du fait de la dilatation du tambour 2. De plus, le dispositif de liaison, comprenant les cardans ainsi que les moyens de coulissement, est agencé pour permettre la rotation du pignon 58 autour du premier et/ou du deuxième axe de flottement 65,66.

La force tangentielle existant au point de contact entre la couronne 45 et le pignon 58 forme, avec la force de réaction de la barre 64, un couple de rappel qui tend à plaquer les dents l'une contre l'autre.

Le système d'entraînement est donc apte à compenser les défauts d'alignement des axes 4, 59 lors de la mise en place, ou suite à la dilatation du tambour.

L'amplitude du déplacement en rotation du pignon 58 autour du premier et/ou du deuxième axe de flottement 65, 66 est inférieure à 10° de part et d'autre de la position de repos.

En variante, le système d'entraînement pourrait être dépourvu des premier et deuxième axes de flottement, le seul degré de liberté du pignon 58 étant alors son mouvement de rotation autour de son axe principal. Ce système d'entraînement, moins robuste, peut néanmoins convenir dans le cas où la dilatation du tambour n'est pas trop importante.

## Revendications

1. Tambour destiné à une installation (1) pour la fermentation aérobie de déchets, comprenant :
- une enveloppe (3) dans laquelle les déchets à traiter sont destinés à être introduits, ladite enveloppe (3) présentant une extrémité amont, une extrémité aval, et un axe nominal (4), ladite enveloppe (3) étant destinée à être entraînée en rotation autour de son axe (4) lors de la fermentation ;
- un bouclier de sortie comprenant une paroi transversale (6) disposée en regard de l'extrémité aval de l'enveloppe (3) ;
- une trappe (13) prévue dans la paroi transversale (6), apte à permettre l'évacuation des déchets fermentés ;
dans lequel la paroi transversale (6) comprend au moins un orifice de ventilation (20, 20a, 20b) distinct de la trappe (13), ledit orifice étant disposé de sorte à permettre une circulation axiale d'air à travers lui, entre l'intérieur et l'extérieur de l'enveloppe ;
**caractérisé en ce qu'**il comprend un dispositif d'obturation (23) de l'orifice de ventilation (20, 20a, 20b), ledit dispositif étant agencé pour pouvoir être déplacé entre une position de fermeture, dans laquelle l'orifice de ventilation est obturé, et une position d'ouverture, dans laquelle l'orifice de ventilation est dégagé.

2. Tambour selon la revendication 1, **caractérisé en ce que** le dispositif d'obturation (23) est maintenu dans sa position de fermeture par des moyens de rappel (29), ledit dispositif d'obturation étant apte à être déplacé vers sa position d'ouverture sous l'action d'une force d'appui exercée à l'encontre de la force des moyens de rappel.

3. Tambour selon la revendication 1 ou 2, **caractérisé en ce que** le dispositif d'obturation (23) comprend au moins un volet (28a, 28b) disposé de sorte à pouvoir obturer, respectivement dégager l'orifice (20, 20a, 20b), lorsque le dispositif d'obturation est en position de fermeture, respectivement d'ouverture, et au moins une tige (24) solidaire du volet (28a, 28b), l'ensemble volet - tige étant monté sur la face extérieure de la paroi transversale (6) de façon à pouvoir coulisser sensiblement radialement.

4. Tambour selon l'une quelconque des revendications 1 à 3, **caractérisé en ce que** la paroi transversale (6) présente au moins deux ensembles (21, 21a, 21b, 21c, 21d) d'au moins un orifice de ventilation (20, 20a, 20b), lesdits ensembles étant répartis sur la surface de ladite paroi transversale (6) et espacés les uns des autres sensiblement régulièrement.

5. Tambour selon la revendication 4, **caractérisé en ce qu'**au moins un ensemble (21) comprend au moins deux orifices (20a, 20b) situés sensiblement sur un même rayon, dans la zone périphérique de la paroi transversale (6).

6. Tambour selon l'une quelconque des revendications 1 à 5, **caractérisé en ce que** la trappe (13) est située dans la zone périphérique de la paroi transversale (6).

7. Tambour selon l'une quelconque des revendications 1 à 6, **caractérisé en ce qu'**il comprend un panneau d'obturation (17) de la trappe (13), monté mobile entre une position de fermeture, dans laquelle il obture la trappe, et une position d'ouverture, dans laquelle la trappe est dégagée.

8. Installation pour la fermentation aérobie de déchets, **caractérisée en ce qu'**elle comprend :
- un tambour (2) selon l'une quelconque des revendications précédentes ;
- des moyens de support (38) du tambour agencés pour que l'axe nominal (4) de l'enveloppe (3) du tambour soit sensiblement horizontal ou légèrement incliné par rapport à l'horizontale ;
- et au moins un système d'entraînement (7) apte à permettre l'entraînement du tambour (2) en rotation autour de son axe (4).

9. Installation selon la revendication 8, **caractérisée en ce qu'**elle comprend une rampe (31) en arc de cercle fixe par rapport au sol, disposée à l'extérieur de l'enveloppe (3), de façon sensiblement concentrique à l'enveloppe, ladite rampe (31) étant apte à coopérer avec un galet (25) prévu sur le dispositif d'obturation (23), de sorte à provoquer le déplacement du dispositif d'obturation de la position de fermeture à la position d'ouverture par appui du galet contre la rampe.

10. Installation selon la revendication 9, **caractérisée en ce que** la rampe (31) est agencée pour coopérer avec le galet (25) lorsque l'orifice de ventilation (20) n'est pas disposé en vis à vis des déchets présents dans le tambour (2).

11. Installation selon la revendication 9 ou 10, **caractérisée en ce que** la rampe (31) comprend une zone centrale (34) sensiblement circulaire, ainsi qu'une zone d'entrée (35) et une zone de sortie (36) aptes à guider le déplacement du dispositif d'obturation (23) entre ses positions d'ouverture et de fermeture.

12. Installation selon l'une quelconque des revendications 9 à 11, **caractérisée en ce que** la rampe (31) est disposée au-dessus du tambour (2), et s'étend au moins sur un quart de la périphérie du tambour (2), entre un plan vertical passant par l'axe du tambour et un plan horizontal passant par l'axe du tambour.

13. Installation selon l'une quelconque des revendications 8 à 12, **caractérisée en ce qu'**elle comprend un dispositif de ventilation permettant la circulation d'air à l'intérieur du tambour (2), le dispositif de ventilation comprenant au moins un ventilateur (12) relié à l'extrémité amont de l'enveloppe (3) du tambour, ledit ventilateur (12) étant agencé pour aspirer et/ou refouler l'air, de sorte que l'air soit introduit, respectivement évacué, au niveau de l'extrémité aval de l'enveloppe (3) de façon sensiblement axiale à travers l'orifice de ventilation (20).

14. Installation selon l'une quelconque des revendications 8 à 13, **caractérisée en ce que** le système d'entraînement (7) comprend un pignon (58) entraîné en rotation autour d'un axe principal (59) sensiblement parallèle à l'axe nominal (4) du tambour (2) et une couronne dentée (45) solidaire du tambour (2), ledit pignon (58) étant disposé de sorte à venir engrener la couronne (45) pour commander la rotation du tambour (2) autour de son axe nominal (4), le pignon (58) étant en outre monté mobile par rapport à un premier axe de flottement (65) distinct de l'axe principal (59) du pignon (58), ledit montage étant agencé pour permettre au moins la rotation libre du pignon autour dudit premier axe de flottement.

15. Installation selon la revendication 14, **caractérisée en ce que** le pignon (58) est en outre monté mobile par rapport à un deuxième axe de flottement (66) distinct de l'axe principal (59) du pignon (58) et du premier axe de flottement (65), ledit montage étant agencé pour permettre au moins la rotation libre du pignon autour dudit deuxième axe de flottement.

## Claims

1. A drum intended for a plant (1) for the aerobic fermentation of wastes, including:
- a casing (3) wherein the wastes to be treated are intended to be introduced, with said casing (3) having an upstream end, a downstream end and a nominal axle (4), with said casing (3) being intended to be driven into rotation about the axle (4) thereof, during the fermentation;
- an outlet shield including a transversal wall (6), positioned opposite the downstream end of the casing (3);
- a manhole (13) provided in the transversal wall (6), suitable for enabling the discharge of the fermented wastes;
wherein the transversal wall (6) includes at least one ventilation hole (20, 20a, 20b) different from the manhole (23), said hole being positioned so as to enable an axial circulation of air therethrough, between the inside and the outside of the casing;
**characterized in that** it includes a closing device (23) for the ventilation hole (20, 20a, 20b), with said device being so arranged as to be able to be moved between a closed position, where the ventilation hole is closed, and an open position, where the ventilation hole is open.

2. A drum according to claim 1, **characterized in that** the closing device (23) is held in the closed position thereof by return means (29), said closing device being suitable for being moved to the open position by the action of a pressure exerted against the force of the return means.

3. A drum according to claim 1 or 2, **characterized in that** the closing device (23) includes at least one shutter (28a, 28b) positioned so as to be able to close, respectively to open, the hole (20, 20a, 20b), when the closing device is in the closed, respectively open position thereof, and at least a rod (24) integral with the shutter (28a, 28b), with the shutter - rod assembly being mounted on the external face of the transversal wall (6), so as to be able to slide substantially radially.

4. A drum according to any one of claims 1 to 3, **characterized in that** the transversal wall (6) includes at least two sets (21, 21a, 21b, 21c, 21d) of at least one ventilation hole (20, 20a, 20b), with said sets being distributed on the surface of said transversal wall (6) and substantially regularly spaced from each other.

5. A drum according to claim 4, **characterized in that** at least one set (21) includes at least two holes (20a, 20b) located substantially on the same radius, in the peripheral area of the transversal wall (6).

6. A drum according to any one of claims 1 to 5, **characterized in that** the manhole (13) is located in the peripheral area of the transversal wall (6).

7. A drum according to any one of claims 1 to 6, **characterized in that** it includes a manhole (13) closing panel (17), mounted to move between a closed position, where it closes the manhole, and an open position, where the manhole is open.

8. A plant for the aerobic fermentation of wastes, **characterized in that** it includes:
- a drum (2) according to any one of the preceding claims,;
- drum supporting means (38) so arranged that the nominal axle (4) of the drum casing (3) is substantially horizontal or slightly sloping with respect to the horizontal;
- and at least a driving system (7) suitable for enabling the driving of the drum (2) into rotation about the axle (4) thereof.

9. A plant according to claim 8, **characterized in that** it includes a rail (31) forming a fixed arc of a circle with respect to the floor, positioned outside the casing (3) substantially concentrically with respect to the casing, said rail (31) being suitable for cooperating with a roller (25) provided on the closing device (23), so as to cause the displacement of the closing device from the closed position to the open position by pressing the roller against the rail.

10. A plant according to claim 9, **characterized in that** the rail (31) is so arranged as to cooperate with the roller (25) when the ventilation hole (20) is not positioned opposite the wastes present in the drum (2).

11. A plant according to claim 9 or 10, **characterized in that** the rail (31) includes a substantially circular central area, as well as an inlet area (35) and an outlet area (36) suitable for guiding the displacement of the closing device (23) between the open position and the closed position thereof.

12. A plant according to any one of claims 9 to 11, **characterized in that** the rail (31) is positioned above the drum (2), and extends on at least a quarter of the drum (2) periphery, between a vertical plane intersecting the drum axle and a horizontal plane intersecting the drum axle.

13. A plant according to any one of claims 81 to 12, **characterized in that** it includes a ventilation device enabling the circulation of air inside the drum (2), with the ventilation device including at least one fan (12) connected to the upstream end of the drum casing (3), said fan (12) being so arranged as to suck in and/or discharge the air, so that the air is substantially axially introduced, respectively discharged, at the downstream end of the casing (3) through the ventilation hole (20).

14. A plant according to any one of claims 8 to 13, **characterized in that** the driving system (7) includes a pinion (58) driven to rotate about a main axle (59) substantially parallel to the nominal axle (4) of the drum (2) and a toothed crown (45) made integral with the drum (2), said pinion (58) being so positioned as to engage the crown (45) in order to control the rotation of the drum (2) about the nominal axle (4) thereof, with the pinion (58) being further mounted to move with respect to a first floating axle (65) different from the main axle (59) of the pinion (58), with said mounting being so arranged as to enable at least the free rotation of the pinion about said first floating axle.

15. A plant according to claim 14, **characterized in that** the pinion (58) is further mounted to move with respect to a second floating axle (66) different from the main axle (59) of the pinion (58) and the first floating axle (65), said mounting being so arranged as to enable at least the free rotation of the pinion about said second floating axle.

## Patentansprüche

1. Trommel für eine Anlage (1) für die aerobe Fermentierung von Abfällen, die folgende Teile umfaßt:
- einen Schacht (3), in den die zu verarbeitenden Abfälle eingeleitet werden sollen, wobei der besagte Schacht (3) ein oberstromiges Ende, ein unterstromiges Ende und eine Nennachse (4) umfaßt, wobei der besagte Schacht (3) dazu bestimmt ist, bei der Fermentierung in Drehung um seine Achse (4) versetzt zu werden;
- einen Ausgangsschild mit einer Querwand (6), die gegenüber dem unterstromigen Ende des Schilds (3) angeordnet ist;
- eine Klappe (13), die in der Querwand (6) vorgesehen und geeignet ist, die Ausleitung der fermentierten Abfälle zu ermöglichen;
in der die Querwand (6) zumindest eine Belüftungsöffnung (20, 20a, 20b) außer der Klappe (13) umfaßt, wobei die besagte Öffnung so angeordnet ist, daß sie eine axiale Luftzirkulation durch sie hindurch zuläßt, und zwar zwischen dem Inneren und dem Äußeren des Schachts;
**dadurch gekennzeichnet, daß** sie eine Vorrichtung für den Verschluß (23) der Belüftungsöffnung (20, 20a, 20b) umfaßt, wobei die besagte Vorrichtung so gestaltet ist, daß sie bewegt werden kann zwischen einer Verschlußposition, in der die Belüftungsöffnung verschlossen ist, und einer Öffnungsposition, in der die Belüftungsöffnung frei ist.

2. Trommel nach Anspruch 1, **dadurch gekennzeichnet, daß** die Verschlußvorrichtung (23) durch Rückhaltemittel (29) in ihrer Verschlußposition gehalten wird, wobei die besagte Verschlußvorrichtung unter Wirkung einer Auflagekraft entgegen der Kraft der Rückhaltemittel in ihre Öffnungsposition versetzt werden kann.

3. Trommel nach Anspruch 1 oder 2, **dadurch gekennzeichnet, daß** die Verschlußvorrichtung (23) zumindest eine Klappe (28a, 28b) umfaßt, die so angeordnet ist, daß die Öffnung (20, 20a, 20b) verschlossen beziehungsweise freigegeben werden kann, wenn die Verschlußvorrichtung in Verschlußposition beziehungsweise Öffnungsposition ist, und zumindest eine Stange (24), die fest mit der Klappe (28a, 28b) verbunden ist, wobei das Ensemble aus Klappe und Stange auf die Außenseite der Querwand (6) so montiert ist, daß es frei radial gleiten kann.

4. Trommel nach einem beliebigen der vorstehenden Ansprüche 1 bis 3, **dadurch gekennzeichnet, daß** die Querwand (6) zumindest zwei Ensembles (21, 21a, 21b, 21c, 21d) mit mindestens einer Belüftungsöffnung (20, 20a, 20b) aufweist, wobei die besagten Ensembles auf der Oberfläche der besagten Querwand (6) verteilt und voneinander deutlich gleichmäßig getrennt sind.

5. Trommel nach Anspruch 4, **dadurch gekennzeichnet, daß** zumindest ein Ensemble (21) zumindest zwei Öffnungen (20a, 20b) umfaßt, die sich deutlich auf einem gleichen Radius in einer peripheren Zone der Querwand (6) befinden.

6. Trommel nach einem beliebigen der vorstehenden Ansprüche 1 bis 5, **dadurch gekennzeichnet, daß** sich die Klappe (13) in der peripheren Zone der Querwand (6) befindet.

7. Trommel nach einem beliebigen der vorstehenden Ansprüche 1 bis 6, **dadurch gekennzeichnet, daß** sie eine Verschlußplatte (17) der Klappe (13) umfaßt, die mobil zwischen einer Verschlußposition, in der sie die Klappe verschließt, und einer Öffnungsposition montiert ist, in der die Klappe frei ist.

8. Anlage für die aerobe Fermentierung von Abfällen, **dadurch gekennzeichnet, daß** sie folgende Teile umfaßt:
- eine Trommel (2) nach einem beliebigen der vorstehenden Ansprüche;
- Stützmittel (38) der Trommel, die so gestaltet sind, daß die Nennachse (4) des Schachts (3) der Trommel deutlich horizontal oder gegenüber der Horizontalen leicht geneigt ist;
- und zumindest ein Antriebssystem (7), das geeignet ist, den Antrieb der Trommel (2) in Drehung um ihre Achse (4) zu ermöglichen.

9. Anlage nach Anspruch 8, **dadurch gekennzeichnet, daß** sie eine Rampe (31) wie ein fixer Kreisbogen im Verhältnis zum Boden umfaßt, die außerhalb des Schachts (3) deutlich konzentrisch zum Schacht angeordnet ist, wobei die besagte Rampe (31) geeignet ist, mit einer auf der Verschlußvorrichtung (23) vorgesehenen Rolle (25) zusammenzuwirken, so daß sie die Verschiebung der Verschlußvorrichtung von der Verschlußposition zur Öffnungsposition durch Drücken der Rolle gegen die Rampe auslöst.

10. Anlage nach Anspruch 9, **dadurch gekennzeichnet, daß** die Rampe (31) gestaltet ist, um mit der Rolle (25) zusammenzuwirken, wenn die Belüftungsöffnung (20) nicht gegenüber den in der Trommel (2) vorhandenen Abfällen angeordnet ist.

11. Anlage nach Anspruch 9 oder 10, **dadurch gekennzeichnet, daß** die Rampe (31) einen zentralen deutlich kreisförmigen Bereich (34) umfaßt sowie einen Eingangsbereich (35) und einen Ausgangsbereich (36), die geeignet sind, die Verschiebung der Verschlußvorrichtung (23) zwischen ihrer Öffnungsposition und ihrer Verschlußposition zu führen.

12. Anlage nach einem beliebigen der vorstehenden Ansprüche 9 bis 11, **dadurch gekennzeichnet, daß** die Rampe (31) über der Trommel (2) abgeordnet ist und sich zumindest über ein Viertel der Peripherie der Trommel (2) zwischen einer vertikalen Ebene, die durch die Achse der Trommel geht, und einer horizontalen Ebene, die durch die Achse der Trommel verläuft, erstreckt.

13. Anlage nach einem beliebigen der vorstehenden Ansprüche 8 bis 12, **dadurch gekennzeichnet, daß** sie eine Belüftungsvorrichtung umfaßt, die die Luftzirkulation im Innern der Trommel (2) ermöglicht, wobei die Belüftungsvorrichtung zumindest einen Ventilator (12) umfaßt, der mit dem oberstromigen Ende des Schachts (3) der Trommel verbunden ist, wobei der besagte Ventilator (12) gestaltet ist, um die Luft anzusaugen und/oder zurückzudrängen, so daß die Luft eingeführt beziehungsweise am unterstromigen Ende des Schachts (3) deutlich axial durch die Belüftungsöffnung (20) herausgeführt wird.

14. Anlage nach einem beliebigen der vorstehenden Ansprüche 8 bis 13, **dadurch gekennzeichnet, daß** das Antriebssystem ein Ritzel (58) umfaßt, das in Drehung um eine Hauptachse (59) versetzt wird, die deutlich parallel zur Nennachse (4) der Trommel (2) verläuft, und einen Zahnkranz (45), der fest mit der Trommel (2) verbunden ist, wobei das besagte Ritzel (58) so angeordnet ist, daß es in den Kranz (45) eingreift, um die Drehung der Trommel (2) um ihre Nennachse (4) zu steuern, wobei das Ritzel (58) ferner im Verhältnis zu einer ersten Schwebeachse (65) mobil montiert ist, die von der Hauptachse (59) des Ritzels (58) getrennt ist, wobei die besagte Montage so gestaltet ist, daß sie zumindest das freie Drehen des Ritzels um die besagte erste Schwebeachse zuläßt.

15. Anlage nach Anspruch 14, **dadurch gekennzeichnet, daß** das Ritzel (58) ferner im Verhältnis zu einer zweiten Schwebeachse (66) mobil montiert ist, die von der Hauptachse (59) des Ritzels (58) und der ersten Schwebeachse (65) getrennt ist, wobei die besagte Montage so gestaltet ist, daß sie zumindest das freie Drehen des Ritzels um die besagte zweite Schwebeachse zuläßt.
